Europäisches Patentamt

**European Patent Office**

Office européen des brevets

(19)

(11) Publication number:

# 0 399 782
A2

(12) ## EUROPEAN PATENT APPLICATION

(21) Application number: **90305544.0**

(51) Int. Cl.5: **A61F 2/18, A61F 2/02**

(22) Date of filing: **22.05.90**

(30) Priority: **23.05.89 US 355327**

(43) Date of publication of application:
**28.11.90 Bulletin 90/48**

(84) Designated Contracting States:
**DE FR GB**

(71) Applicant: **MINNESOTA MINING AND MANUFACTURING COMPANY**
**3M Center, P.O. Box 33427**
**St. Paul Minnesota 55133(US)**

(72) Inventor: **Scholz, Matthew T., c/o Minnesota Mining and**
**Manuf. Co., 2501 Hudson Road, P. O. Box 33427**
St. Paul, Minnesota 55133-3427(US)
Inventor: **Stypulkowski, Paul H., c/o Minnesota Mining and**
**Manuf. Co., 2501 Hudson Road, P. O. Box 33427**
**St. Paul, Minnesota 55133-3427(US)**
Inventor: **Muchow, David C., c/o Minnesota Mining and**
**Manuf. Co., 2501 Hudson Road, P. O. Box 33427**
**St. Paul, Minnesota 55133-3427(US)**

(74) Representative: **Baillie, Iain Cameron et al**
**c/o Ladas & Parry Isartorplatz 5**
**D-8000 München 2(DE)**

(54) **Modifying a membrane for use as a graft.**

(57) Disclosed is a cohesive membrane (1) useful in tissue grafting having at least one perforation therein, which preferably further has a handle (2) projecting angularly at least about 500 μm from one surface of the membrane. The invention relates particularly to tympanic membrane repair.

FIG. 1

EP 0 399 782 A2

## MODIFYING A MEMBRANE FOR USE AS A GRAFT

The present invention relates to a method for modifying a membrane intended for use as a surgical graft. In particular, the present invention relates to modifying a membrane for use in tympanic membrane repair.

Membranes formed from both natural and synthetic substances are known for use as implants and grafts for repairing damaged natural tissue. For example, in the surgical repair of perforated eardrums (i.e., tympanoplasty), a replacement membrane is placed on the damaged eardrum to cover the perforation and the ear packed with surgical sponge material to hold the graft in place. In time, the natural tissue response hopefully incorporates the graft into the surrounding tissue.

Because of the low adhesion properties of many materials useful in tympanoplasty, problems can arise both during and after the surgical procedure. During the surgery itself, accurate placement of the graft is often difficult, the slippery nature of the replacement membrane making it difficult to handle. Furthermore, even though the ear is packed with sponge material to keep the replacement membrane in place, gross movements of the head may cause slippage of the material with respect to the perforation. Even the packing procedure itself may dislodge the replacement membrane from its proper position. Also, incorporation of the replacement membrane into the surrounding natural tissue is slow and often incomplete.

Accordingly, the present invention is a method for texturizing a replacement membrane for use in tissue repair comprising perforating the membrane. One or more perforations in the membrane permit natural tissue growth inside the perforation, thereby anchoring the membrane to the surrounding natural tissue. Membrane material projecting from its surface as a result of the perforation process also serves to mechanically anchor the membrane in place on the natural tissue. The present invention is also a method of modifying a replacement membrane for use in tissue repair comprising providing the membrane with a handle that projects from the surface of the membrane. When the membrane is used to repair a perforated eardrum, the handle provides a convenient means for inserting the membrane through the perforation and accurately positioning it.

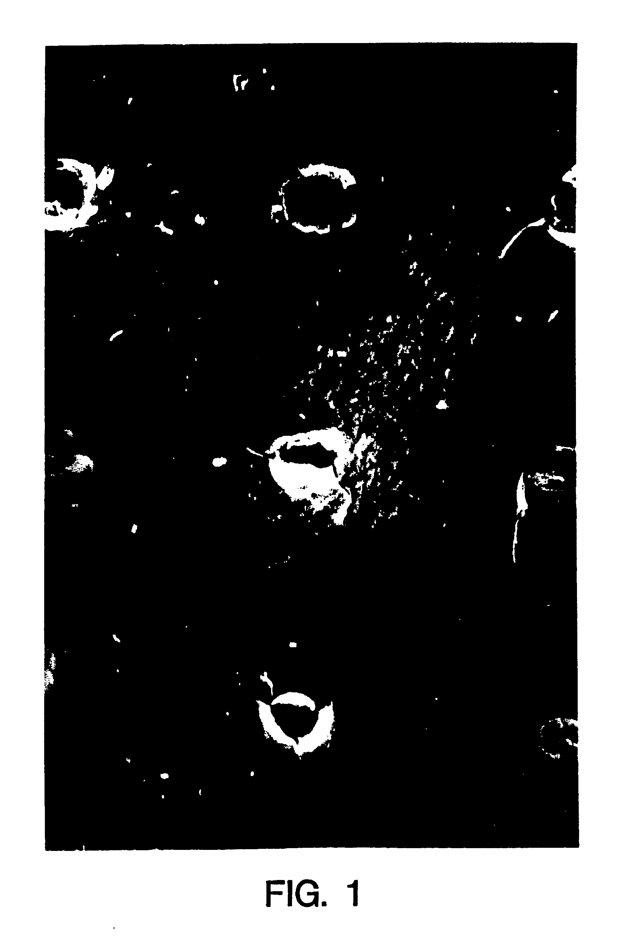

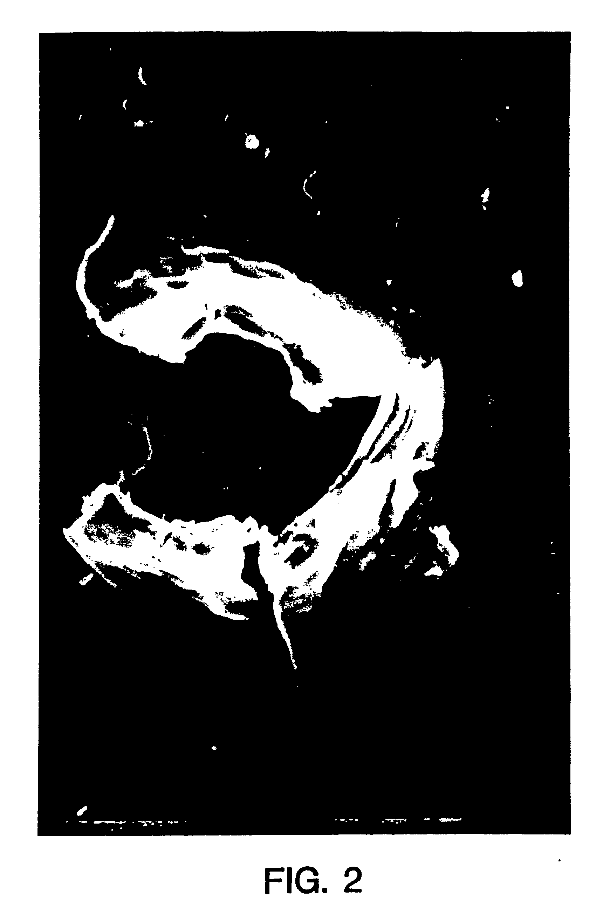

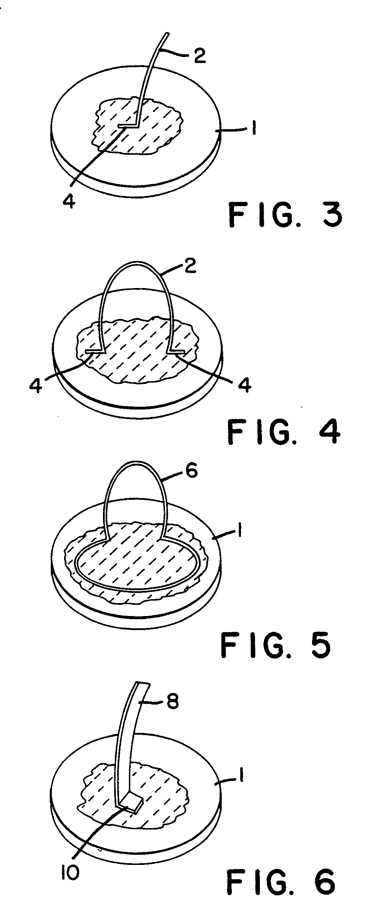

Fig. 1 is an electron micrograph showing a membrane of the present invention magnified 500x, and Fig. 2 is an electron micrograph showing a perforated membrane of the present invention magnified 3,000x. Figs. 3, 4, 5, and 6 are perspective views of different embodiments of the membrane having a handle according to the present invention.

Any cohesive membrane useful in natural tissue repair is useful in accordance with the present invention. Such membranes include transplanted tissue from the recipient (i.e., an autograft), transplanted tissue from an individual of the same species (i.e., a homograft), or transplanted tissue from an individual of another species (i.e., a xenograft). In addition to natural tissue, which can be glutaraldehyde treated, such as fascia, fat, perichondrium, and cartilage, synthetic materials are also useful, such as polylactic acid, silicone, polyurethanes, dacron, and polytetrafluoroethylene. Preferably, the membrane is an artificial membrane comprising a product made by cross-linking molecules of interpenetrating denatured collagen coupled at their lysine epsilon amino groups with a coupler through carbonyl groups, sulfonyl groups, or combination thereof on the coupler wherein non-coupled lysine epsilon amino groups are bonded to a modifier wherein the modifier is a carbonyl, sulfonyl, carbamoyl, or $\beta$-malic acid group. The preferred membrane used in accordance with the present invention is made by denaturing coupled, and preferably modified, collagen molecules. The coupled and modified collagen molecules useful in accordance with the present invention and their method of manufacture are disclosed in United States Patents 4,713,466 and 4,883,864. The carbonyl groups, sulfonyl groups, or combination thereof on the coupler are bonded together through an R group wherein R is a $C_{2-20}$ saturated or unsaturated aliphatic, aromatic, or aliphatic-aromatic group that is unsubstituted or substituted with halogen, or $C_{1-4}$ carboxy, alkyl, or alkoxy and having 0-5 heteroatoms wherein the heteroatom is oxygen, sulfur, or nitrogen. Preferably, the coupler has the formula $-CO-CH_2-CH_2-CO-$ or $-CO-CH_2-CH_2-CH_2-CO-$. The modifier has the formula $RCO-$, $RNHCO-$, $RSO_2-$, or $COOR'CHOHCH(COOR')-$ wherein R is a $C_{2-20}$ saturated or unsaturated aliphatic or aromatic group that is unsubstituted or substituted with halogen, $C_{1-4}$ alkyl or alkoxy, and having 0-5 heteroatoms wherein the heteroatom is oxygen, sulfur, or nitrogen, and $R'$ is H, Na, K, or Li. Preferably, the modifier has the formula $R-NH-CO-$, more preferably $CH_3(CH_2)_3-NH-CO-$. Coupling is performed by reacting native collagen with a poly-functional amine-reactive agent selected from the group consisting of a carboxylic acid halide, sulfonyl halide, anhydride, and reactive active ester in aqueous media at a pH greater than about 8 and at a temperature between 0 and 35° C. Preferably, the poly-functional amine-reactive agent is succinic acid dichloride or glutaric acid dichloride. Preferably, the ratio of poly-functional amine-reactive agent used per weight of native collagen varies between about 1/100 and 6/1, more preferably between about 1/50 and 2/1. Modification involves reacting the native collagen molecules with a mono-functional amine reactive agent selected from the group consisting of an anhydride, acid halide, sulfonyl halide, active ester, isocyanate, and epoxy succinic acid in aqueous media at a pH greater than about 8 and at a temperature between about 0 and 35 °C. Preferably, the mono-functional amine-reactive agent is n-butyl isocyanate or epoxy succinic acid. The weight ratio of mono-functional amine-reactive agent used per amount of native collagen is between about 1/100 and 10/1, more preferably between about 1/10 and 1/1. Coupling and modifying are performed in any order, or simultaneously.

Denaturing is preferably performed by heating the coupled and preferably modified collagen molecules in aqueous media or non-aqueous media at a temperature between about 40 and 120 °C. Heating causes the normal collagen helix to unwind, producing single stranded collagen molecules coupled at their lysine epsilon amino groups. Upon cooling, the solution forms a gel, which is believed to contain an interpenetrating network of hydrogen bonded $\alpha$-helixes with segments of single stranded collagen exposed.

Accordingly, the heated collagen molecules are cast into a desired shape, such as a film, through the use of an appropriate mold and then allowed to cool and gel. Preferable thicknesses for the gel are between about 0.127 cm and 1.27 cm, more preferably between about 0.254 cm and 0.613 cm. After cooling, the interpenetrating, denatured collagen molecules are cross-linked to form an artificial membrane useful in tympanic membrane repair.

Preferably, the gel is dehydrated, which is believed to cause some cross-linking of the collagen molecules. After dehydration, the membrane has a preferable thickness between about 0.1 and 0.5 mm, more preferably between about 0.15 and 0.25 mm. Preferably, after dehydration the molecules are further cross-linked to increase the burst strength of the membrane. Further cross-linking is preferably performed by treating the membrane with chemical cross-linking agents or exposing the membrane to sufficient actinic radiation. Useful cross-linking agents include polyfunctional amine reactive agents such as a carboxylic acid halide, sulfonyl halide, anhydride, and reactive ester. Examples of such agents are disclosed in the aforementioned United States Patent 4,713,466. Methods for using chemical cross-linking agents will be apparent to the skilled artisan. Preferably, chemical cross-linking is performed using non-aqueous systems in order to prevent hydrolysis of the cross-linking agent. For example, the membrane is immersed in succinyl chloride, either neat or in pyridine or other suitable organic base that would neutralize HCl evolved during the cross-linking reaction, at an amount of about 0.001-0.1 moles of agent per gram of membrane, preferably about 0.005-0.05 moles/g. Alternatively, chemical cross-linking can be carried out in aqueous media while maintaining a pH of 8-10 and using an amount of cross-linking agent between about 0.05 and 0.5 moles per gram of membrane, depending on the rate of hydrolysis of the particular agent used. Useful forms of actinic radiation include ultraviolet light, gamma radiation, and electron beam radiation. Sources and methods of applying radiation to the membrane will be apparent to the skilled artisan. After cross-linking, the membrane is preferably washed to remove unreacted agents, and further sterilized, e.g., by autoclaving or exposure to gamma radiation or ethylene oxide, before use in tympanic membrane repair.

The artificial membrane preferred for use in accordance with the present invention is optionally cleaned and purified before use. For example, either before or after heating, but prior to cooling, a solution of the coupled and modified collagen molecules are filtered to remove particles. At any stage in the process after denaturation, extraction purification, e.g., using sterile water for injection, high-purity grade acetone, or other suitable solvent or solvent mixture, can be employed.

The size of the membrane itself will vary depending upon its intended use. When used in human tympanic membrane (eardrum) repair, the membrane has a preferable thickness between about 20 and 500 $\mu$m, more preferably between about 45 and 155 $\mu$m, and length and width dimensions slightly larger than the perforation in the eardrum, which preferably effects a surface area on one side of the membrane between about 3 and 500 mm$^2$. When a hydratable material is used as the membrane, the foregoing ranges are determined when the membrane is fully hydrated, i.e., after immersion in physiological buffer at 37 °C until equilibrium is reached. Perforations are made in the membrane in accordance with the present invention by a variety of means using devices that will be apparent to the skilled artisan. For example, the membrane can be initially cast in a dish having holes through which spikes project upwardly. After the membrane forms in the dish, the spikes are withdrawn from the holes leaving the perforated membrane. For thermoplastic membranes a heat-staking process (using hot metal wires) can be used to create the perforations. Preferably, the membrane is perforated after it is formed by forcing it over a matrix of pyramidal spikes attached to a plate. By perforating the membrane in this manner, the rough edges created around the perforations in the membrane, as shown in Figs. 1 and 2, act like hooks to adhere the membrane to the tissue surface. The size, number, and arrangement of the perforations vary based on the considerations of tissue strength, desirability of water and air impermeability, and creation of angular edges for adhering the membrane to the tissue surface.

Preferably, the perforations have a minimum cross dimension greater than about 50 $\mu$m, and a maximum cross dimension less than about 400 $\mu$m. More preferably, the spikes are designed and pressure is applied to the membrane such that holes that are large enough to permit fibroblast migration and reproduction, yet small enough to permit the membrane to act as a water barrier, are made in the membrane. Accordingly, the holes are more preferably made in the membrane to have a minimum cross dimension greater than about 140 $\mu$m, which is designed to accommodate optimum rates of fibroblast migration and reproduction, and a maximum cross diameter less than about 250 $\mu$m, which permits the membrane to act as a water barrier in the absence of pressure. Any number of perforations are present in the membrane, and they are arranged on the surface of the membrane in any desired pattern. Preferably, the perforations are arranged in a square grid about 200-600 $\mu$m apart as measured from the center of the perforation, more preferably between about 300 and 500 $\mu$m apart. As shown in Figs. 1 and 2, the perforations have a size of about 150 $\mu$m, and are located about 500 $\mu$m apart in a square grid pattern. Preferably, the edges of the perforations have a height, i.e., the distance projecting from the surface of the membrane, between about 25 and 500 $\mu$m, more preferably between about 50 and 150 $\mu$m, most preferably about 100 $\mu$m.

The handle provided on the membrane in accordance with the present invention has various forms, is made of various tissue-compatible materials, and is attached to the membrane in various ways depending upon the surgery involved, membrane material used, and surgical tools employed. Preferably, the handle is made of a biodegradable material. Useful biodegradable materials include polymers having at least one hydrolyzable ester, amide, or ester/amide linkage, such as polylactic acid, polygalactic acid, lactic acid/galactic acid copolymers, polyhydroxybutyric acid, polydioxanone, collagen, collagen derivatives, catgut derivatives, polyurethanes, polytetrafluoroethylene, glutaraldehyde-treated tissue, and silicones. For example, the handle can be medical grade suture thread that is passed through the membrane and back again; by pulling on both ends of the thread the membrane can be held in place, and by pulling on one end of the thread, the handle can be removed from the membrane after surgical implantation. When using the preferred membrane in accordance with the present invention, a portion of a suture thread is advantageously set in the denatured collagen solution such that a useful length of the thread projects axially from the center of the cooling solution while a sufficient portion of the thread is imbedded in the solution to provide an anchor once the membrane is formed. After membrane formation, the suture is then an integral part of the membrane. Suture handles are exemplified in Figs. 3, 4, and 5. As shown in Fig. 3, one end 4 of suture thread 2 is imbedded in membrane 1. Referring to Fig. 4, both ends 4 of suture thread 2 are imbedded in membrane 1 to form the handle as a closed loop. A closed-loop handle is also shown in Fig. 5, wherein part of continuous suture thread 6 is imbedded in the membrane 1. Instead of a suture thread, as shown in Fig. 6, the handle can also be made of a strip 8 of the same material as the membrane, the end 10 of which is imbedded in the membrane 1. Alternatively, the membrane and handle are cast in one piece from the same material to form a unitary structure, i.e., a mold the shape of the membrane and handle is used.

As shown in the accompanying figures, the size and shape of the handle vary. Generally, it is an elongated strip of material having a length, i.e., the distance projecting from the surface of the membrane, between about 100 and 2000 $\mu$m, more preferably between about 150 and 1000 $\mu$m. The handle can be located on any part of the lateral surface of the membrane, but is preferably located as close to the center of the membrane as possible.

To more clearly describe the present invention, the following, non-limiting examples are provided. All parts and percentages in the examples are by weight unless indicated otherwise.

## EXAMPLE 1

A membrane is prepared using modified collagen. Coupled and modified collagen is prepared by addition of a chemical coupling agent and subsequently an amine modifying agent, using aseptic technique under a laminar flow hood. About 500 ml of chilled (4° C) vitrogenTM collagen Type I solution (Collagen Corp., Palo Alto, CA) is poured into a glass reactor vessel. The pH of the solution is brought to 9 by the addition of 5N and 1N sodium hydroxide. At a temperature between about 4 and 8° C, the solution is vigorously agitated and 0.28 g of succinyl chloride is added all at once to the solution. The reaction is allowed to proceed for 20 minutes, and during this time the pH is held within the range between 9.0 and 9.35 by the gradual addition of 1N sodium hydroxide solution as needed.

The product obtained above is modified with a reagent that reacts with the exposed amine groups on the coupled collagen molecules. The vigorous agitation of the solution is continued while 0.35 g of neat butyl isocyanate is added to the vessel as rapidly as possible. The reaction is allowed to proceed for 1 hour, and during this time the pH is held within the range between 9.0 and 9.25 by the addition of 1N sodium hydroxide solution as needed. As the reaction proceeds, the solution is gradually allowed to warm to room temperature. The pH of the solution is then decreased slowly by the addition of 6N hydrochloric acid to precipitate out the modified collagen. The acid is added until the cloudiness of the solution stops increasing (generally at a pH of about 4.0-4.7). The solution is allowed to continue mixing for 5 minutes. The resulting collagen slurry is centrifuged at a temperature of about 4°C, at a speed sufficient to create a force of about 10,000 G (as measured at the bottom of the centrifuge tube), and the supernatant removed.

The resulting collagen precipitate is washed by adding pyrogen free water to a total volume of about 240 ml of collagen suspension, and the pH is adjusted to within the range of 4.5 and 4.7 by the addition of 1N hydrochloric acid or 1N sodium hydroxide as needed. The neutralized collagen suspension is centrifuged at 4°C, at a speed of about 10,000 rpm (16,000 G at the bottom of the centrifuge tube), for 10 minutes. After removing the supernatant, this procedure is repeated three times for a total of four washings. The final collagen concentration is adjusted to approximately 2% by weight.

The washed and modified collagen product is then denatured by heating at 60°-80°C for one hour in a water bath. The material is then neutralized with 1N sodium hydroxide as needed to bring the pH to within the range of 7.0 to 7.2.

About 6.0 ml of the warm denatured collagen solution is transferred into a sterile flat-bottom polytetrafluoroethylene dish 6 cm in diameter. The side of which has been machined to about a 5-15° angle outward from the bottom to facilitate subsequent membrane removal, and the bottom of the dish has been machined roughly to create a relief of about a 1.27 μm in the surface of the membrane. The dish is then covered with a sterile petri dish and allowed to sit at ambient conditions (approximately 23°C) for 25 minutes to allow the denatured collagen to slowly cool and gel.

The covered dish is then transferred to a prepurged, nitrogen box having about 70 l total volume. At a nitrogen flow rate of about 12-15 1/minute, the material is dried for about 24-36 hours to form a dehydrated membrane about 0.058 mm thick.

The membrane is laid on an aluminum foil at a distance of 15-16 cm from a 15 Watt, 254 nm, ultraviolet light source for a period of about 4 hours.

The membrane is then purified by immersion in 150 ml of high-purity grade acetone and allowed to sit for a minimum of 2 hours under gentle agitation. The charge of acetone is then decanted and the purification/extraction step repeated twice.

The membrane is used for repairing a 3 mm perforation in the tympanic membrane of a chinchilla. After the membrane is wetted with sterile, pyrogen-free water and allowed to hydrate for 5 minutes to facilitate cutting, a 1 cm diameter disk is then cut from the membrane using a stainless steel punch die and a small arbor press cutting against a polytetrafluoroethylene plate. The disk is then texturized to create perforations in the membrane by forcing it over pyramidal titanium spikes fixed 500 μm apart in a square pattern on a plate, each spike being about 150 μm$^2$ at its base and about 150 μm high. The disk is then allowed to dry for at least one hour. After drying the membrane is sealed in appropriate packaging and sterilized by exposure to 0.5-0.6 Mrad gamma radiation.

Surgical repair is commenced by making a posterior approach to the bulla of the anesthetized chinchilla. The margin of the perforation in the tympanic membrane is neatened and a region 1.5 mm wide is denuded of epithelium using a half-Hough tool. The artificial membrane disk is removed from its sterilized packaging and trimmed to an appropriate diameter, i.e., such that at least 3.4 mm of the membrane contacts the intact borders of the ruptured eardrum, or about 1.5 times the diameter of the eardrum perforation. The trimmed membrane is positioned over the perforation so that the side of the disk bearing the raised areas formed during texturizing faces the eardrum tissue. Ten minutes are allowed for fibrin formation to completely proceed within the perforations on the disk. Although no sponge packing of the bulla is used, gross movements of the animal performed between about 10 minutes and one hour after surgery do not displace the disk. Tympanometry is performed after several days using a model 6A typanometer (Maico, Minneapolis, MN), which shows near normal tympanic membrane function. A histological evaluation of the implants removed after 30 days shows fibroblast infiltration of the areas, as well as the deposition of new type I collagen within the graft matrix.

EXAMPLE 2

A texturized disk prepared as in EXAMPLE 1 is used to close a 2.5 mm perforation in the tympanic membrane of a monkey. An approach across the ear canal is made to the damaged tympanic membrane of the anesthetized monkey, the margin of the perforation is neatened, and the epithelium peeled back around its circumference. The disk is trimmed and positioned over the perforation from the middle ear side as in EXAMPLE 1. The edges of the epithelium are then folded over the top of the disk and ten minutes are allowed for fibrin formation to occur within the perforations. Gross movements of the animal do not dislodge the disk, even in the absence of sponge packing.

## EXAMPLE 3

A membrane prepared as in EXAMPLE 1, without perforation, to have a thickness of about 100 $\mu$m is cut to make a strip about 1 mm long and 0.25 mm wide, which is then bent and creased in the middle at a 90° angle to form a handle. One leg of the handle is imbedded into a collagen solution that is prepared and cast as in EXAMPLE 1 such that the other leg projects at a right angle from the solution. While maintaining the handle in this position, the solution is allowed to gel and then dried, irradiated, and purified as in EXAMPLE 1. The completed membrane can then be cut to a desired size to form a membrane with a handle, such as shown in Fig. 5.

## Claims

1. A cohesive membrane useful in tissue grafting having at least one perforation therein wherein the perforation has a minimum cross dimension greater than about 50 $\mu$m and a maximum cross dimension less than about 400 $\mu$m.

2. The membrane of claim 1 wherein the perforation has edges projecting from the membrane.

3. The membrane of claim 1 wherein the film has a thickness between about 0.1 and 0.5 mm.

4. The membrane of claim 2 further having a handle projecting at least about 500 $\mu$m from one surface of the membrane.

5. A non-toxic membrane useful in tissue grafting having a handle projecting at least 500 $\mu$m from one surface of the membrane.

6. The membrane of claim 5 wherein the handle comprises a suture, the ends of which are anchored in the membrane.

7. A method comprising the step of perforating a non-toxic membrane useful in tissue grafting.

8. The method of claim 7 comprising perforating the membrane to obtain at least one perforation having a minimum cross dimension greater than about 50 $\mu$m and a maximum cross dimension less than about 400 $\mu$m.

9. The method of claim 7 wherein perforating is effected using parallel, pyramidal spikes projecting from a plate and located between about 200 and 500 $\mu$m apart.

10. Use of a membrane in preparing a membrane for repairing the eardrum of a mammal by surgically applying a membrane to the eardrum wherein the membrane comprises the membrane of any one of claims 1-6.

FIG. 1

FIG. 2

FIG. 3

FIG. 4

FIG. 5

FIG. 6